# EUROPEAN PATENT SPECIFICATION

(11) **EP 1 886 706 B1**
(45) Date of publication and mention of the grant of the patent: **19.01.2011**
(21) Application number: 06118580.7
(22) Date of filing: 08.08.2006
(51) Int. Cl.: A61M 16/20, F16K 17/04, A62B 9/02

(54) **Pressure limiting valve, particularly for medical assisted-ventilaton equipment**
Druckbegrenzungsventil, im Besonderen für medizinisches Gerät zur assistierten Beatmung
Valve de limitation de pression, en particulier pour un équipement médical de ventilation assistée

(43) Date of publication of application: 13.02.2008
(73) Proprietor: Teleflex Medical Europe Ltd, Westmeath (IE)
(72) Inventor: Bergamaschi, Paolo, 41033 Concordia sulla Secchia MO (IT)
(74) Representative: Modiano, Micaela Nadia

(56) References cited:
- EP-A1- 0 923 959
- GB-A- 1 593 871

## Description

The present invention relates to a pressure limiting valve, particularly for medical assisted-ventilation equipment.

In emergency conditions, such as for example first-aid actions or during the transport of a patient in an ambulance or other vehicles, it is often necessary to support or assist the spontaneous ventilation of the patient by means of assisted-ventilation techniques performed by health workers.

One of the possible assisted-ventilation techniques is performed by using a bag made of elastic material, which is provided with an intake and a delivery; the intake is associated by means of a duct to a source for supplying air and oxygen under pressure, and the delivery is associated by means of a connector to a device for leading to the respiratory system of a patient, such as a mask or a tracheal tube.

Air is introduced in the patient by a physician or by a health worker by applying pressure to the elastic bag, while the subsequent expiration step occurs due to elastic return of the lungs.

To achieve good ventilation, it is necessary to apply a positive internal pressure to the lungs so as to achieve their extension and facilitate gaseous exchange within the alveoli. The injection pressure required for correct ventilation depends on the build of the patient, on his age and on his clinical condition.

If the value of the pressure fed into the pulmonary cavities is excessively high, or if excessively sudden variations thereof occur, barotraumas, i.e., lesions of the pulmonary tissues due to the consequent lack of equilibrium between pressure inside and outside the pulmonary cavities, can occur.

In order to obviate these drawbacks, it is known to use pressure limiting valves applied at a connector which is interposed between the delivery of the bag and the mask or tracheal tube and provided with a vent.

The valve limits the inflow pressure according to the physical or clinical characteristics of the patient.

These valves limit the air inflow pressure but do not limit the volume of the air, which is instead determined by the supply source.

Two kinds of pressure limiting valve used in these devices for assisted ventilation are known.

A first type of valve comprises a body in which an intake port and a vent port are formed; a flat element is accommodated within the body and is kept in a position for closing the intake port by a helical spring made of metal, which is conveniently preloaded. The valve opens when the supplied air pressure is higher than a threshold value determined by the resistance of the spring.

Means for adjusting the load of the spring are provided, and by acting on said means it is possible to modify the threshold value of the pressure above which the valve opens.

This known type of pressure limiting valve has drawbacks, including the fact that it is not possible to use them in association with diagnostic equipment whose operation is based on the use of magnetic fields: the spring that keeps the flat element in the closed position, being made of metallic material, in fact has a deforming action on the magnetic field.

Another drawback is that said valves are structurally complicated, since they are constituted by numerous components and have significant construction costs.

A second type is constituted by a valve of the faucet type, which comprises a cylindrical jacket provided with an intake port and a discharge port and inside which a hollow cylindrical body is accommodated so that it can rotate and substantially snugly, said body being provided with a partition for blocking the venting port which has an inclined contour. A larger or smaller opening of the venting port corresponds to different angular positions of the cylindrical body and therefore of the corresponding partition.

Although said valve is simple and cheap to manufacture and allows, by means of the angular positioning of the flow control partition, to adjust the maximum insufflation pressure, it does not however allow high precision in adjustment and exposes the patient to the danger of severe lung damage if the venting port is accidentally left in the fully closed position, therefore requiring adequate preparation of physicians and/or health workers.

<Pressure valves with adjustment features are known from GB 1 593 871 and EP 0 923 959.>

The aim of the present invention is to eliminate the above mentioned drawbacks of known valves, by providing a pressure limiting valve particularly for medical assisted-ventilation equipment which allows safe use for the patient.

Another object of the present invention is to allow accurate adjustment of the maximum pressure threshold value at which the valve opens, so as to adapt it to the characteristics of the patient.

Another object of the present invention is to ensure use of the valve even in combination with the use of diagnostic equipment which uses a magnetic field, such as for example magnetic-resonance equipment.

Within this aim, an object of the present invention is to provide a valve which has a simple structure, is relatively easy to provide in practice, safe in use, effective in operation and has a relatively low cost.

This aim and these and other objects, which will become better apparent hereinafter, are achieved by a pressure limiting valve, according to the present invention, that has the features set forth in claim 1.

Further characteristics and advantages of the present invention will become better apparent from the following detailed description of a preferred but not exclusive embodiment of a pressure limiting valve, particularly for medical assisted-ventilation equipment, illustrated by way of non-limiting example in the accompanying drawings, wherein:

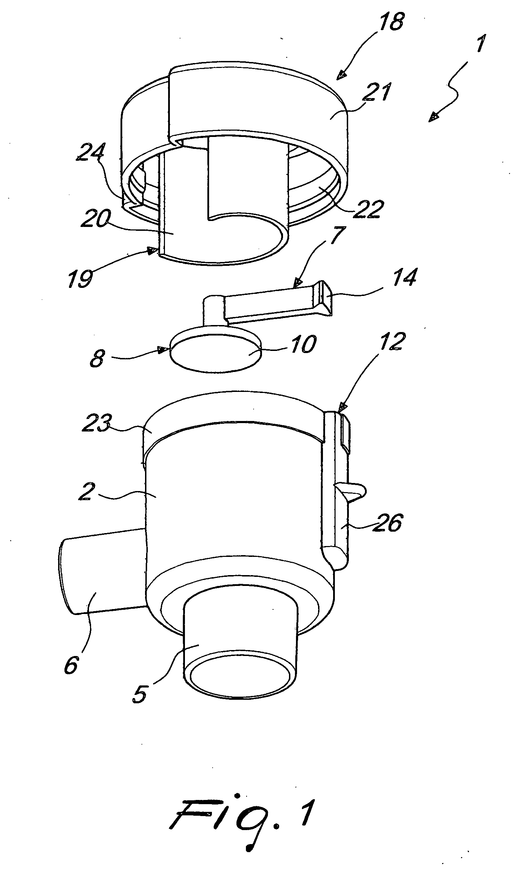

Figure 1 is an axonometric exploded view of the pressure limiting valve according to the invention;

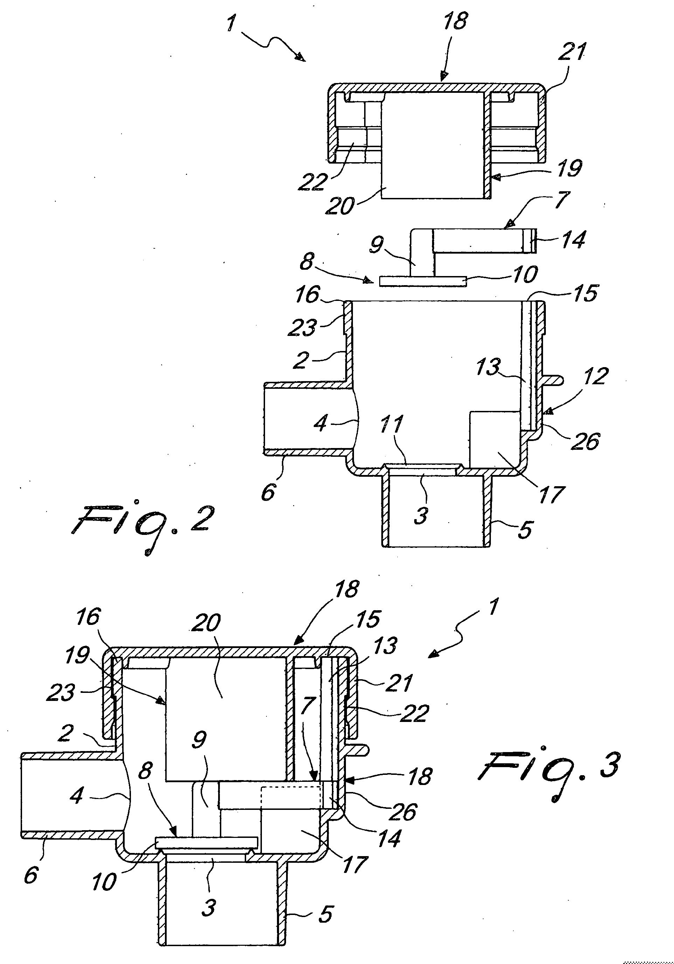

Figure 2 is a sectional exploded view of the pressure limiting valve according to the invention;
Figure 3 is a sectional view of the pressure limiting valve according to the invention;
Figures 4 and 5 are two sectional plan views of the pressure limiting valve according to the invention, in two different operating configurations, in which the pressure threshold value is respectively equal to a maximum value and to a minimum value.

With reference to the figures, the reference numeral 1 generally designates a pressure limiting valve.

The valve 1 comprises a hollow body 2, which is provided with an intake port 3 and a discharge port 4 for a fluid.

In the embodiment shown and with particular reference to the use of the valve 1 in the field of assisted-ventilation equipment, the valve 1 can be connected by means of an intake connector 5, arranged at the intake port 3, to a source for the external supply of the fluid, which is constituted generally by a gaseous compound of air and oxygen. The discharge port 4 of the gaseous compound is instead formed by a vent 6.

In particular, the hollow body 2 is substantially cylindrical with a circular transverse cross-section, at one end of which the intake opening 5 is formed, said body being open at the opposite end.

The vent 6 is tubular and is arranged on the side wall of the hollow body 2 with an arrangement which is substantially perpendicular to the intake opening 5.

The valve 1 further comprises a flat spring 7, which is accommodated within the hollow body 2 and has an end which is rigidly associated therewith and supports a flow control element 8 for the intake port 3.

The flat spring 7 has a constant transverse cross-section and is arranged substantially at right angles to the fluid inflow stream. The flat spring 7 can further flex in the direction in which the flow control element 8 moves away from the intake port 3 at a presettable threshold value of the pressure applied by the fluid at the intake port 3.

In particular, the flat spring 7 is supported in a cantilever fashion by the hollow body 2 and the flow control element 8 is rigidly associated proximate to the free end thereof.

Advantageously, the flat spring 7 and the flow control element 8 are provided monolithically, for example by thermoforming a plastic material of the elastically deformable type, such as silicone or polyurethane.

The flat spring 7 has a tab 9 for supporting the flow control element 8. In particular, the flow control element 8 is constituted by a flat element 10 which is substantially circular, and the tab 9 is constituted by a tubular element which is arranged substantially at right angles respectively to the longitudinal axis of the flat spring 7 and to the plane of arrangement of the flat element 10.

The flat element 10 can be engaged hermetically in a seat 11 which is formed at the intake port 3 and is substantially annular and flared so as to converge toward the intake port 3.

Means for coupling to the hollow body 2 the end of the flat spring 7 that lies opposite the flow control element 8 are provided.

Said coupling means comprise a guide 12, which has substantially a prism-like dovetail shape and runs substantially longitudinally with respect to the hollow body 2.

In particular, the guide 12 comprises a groove 13, with which an anchoring end 14 of the flat spring 7 mates which lies opposite the end that supports the flat element 10 and can be inserted in the groove 13 through an opening 15 provided proximate to the edge 16 of the hollow body 2 on its open end.

In any case, alternative embodiments of the coupling means are not excluded.

Two side walls 17 for containing the flat spring 7 protrude from the longitudinal edges of the guide 12, are substantially parallel to each other and flank along at least one portion two opposite sides of the flat spring 7. The side walls 17 guide the flexing of the spring along a direction for moving the flat element 10 away or toward the intake port 3, avoiding lateral inflections thereof.

The valve 1 further comprises means 18 for adjusting the pressure threshold value. The adjustment means 18 comprise elements for adjusting the flexibility of the flat spring 7.

In particular, the adjustment elements comprise an abutment element 19, which is adapted to make contact in at least one point or transverse line with the face of the flat spring 7 that lies opposite the face that supports the flat element 10 and is associated with the hollow body 2, which can move by sliding along the length of the flat spring 7.

The movement of the abutment element 19 along the longitudinal axis of the flat spring 7 allows a variation of the length of the useful portion thereof formed between the end for supporting the flap 10 and the point or transverse line of contact, consequently varying the flexibility of the spring.

In particular, a movement of the abutment element 19 toward the flat element 10 leads to a reduction of the longitudinal size of said useful portion, consequently reducing the flexibility of the flat spring 7 and increasing the corresponding pressure threshold value; a movement of the abutment element 19 away from the flat element 10 leads to an increase in the longitudinal measurement of the useful portion, with a consequent increase in the flexibility of the flat spring 7 and a consequent decrease of the pressure threshold value.

In particular, the abutment element 19 is constituted by a spiral wall 20, which is rotatably associated with the hollow body 2.

The spiral wall 20 is fixed, at its end which lies opposite the one suitable to make contact with the flat spring 7, to the internal face of an annular element 21, which is rotatably connected to the hollow body 2 at the open end thereof.

The annular element 21 has an annular collar 22, which protrudes toward the center on its internal surface. A ring 23 for fixing the annular element 21 is formed so as to protrude toward the center on the outer surface of the hollow body 2 and proximate to the edge 16. The annular collar 22 engages below the fixing ring 23, avoiding the extraction of the annular element 21.

Means are provided for stopping the rotation of the annular element 21 in two configurations for adjusting the valve 1 at maximum and minimum pressure threshold value limit.

In particular, the annular element 21 has a cylindrical shape with a substantially circular cross-section, and said stop means comprise at least one portion of its side wall which protrudes inward and a first tooth 24 and a second tooth 25 formed at its ends. The rotation of the annular element 21 with respect to the hollow body 2 is limited by the contact of the first tooth 24 and the second tooth 25 with a protrusion 26 on the outer surface of the hollow body 2 at the guide 12.

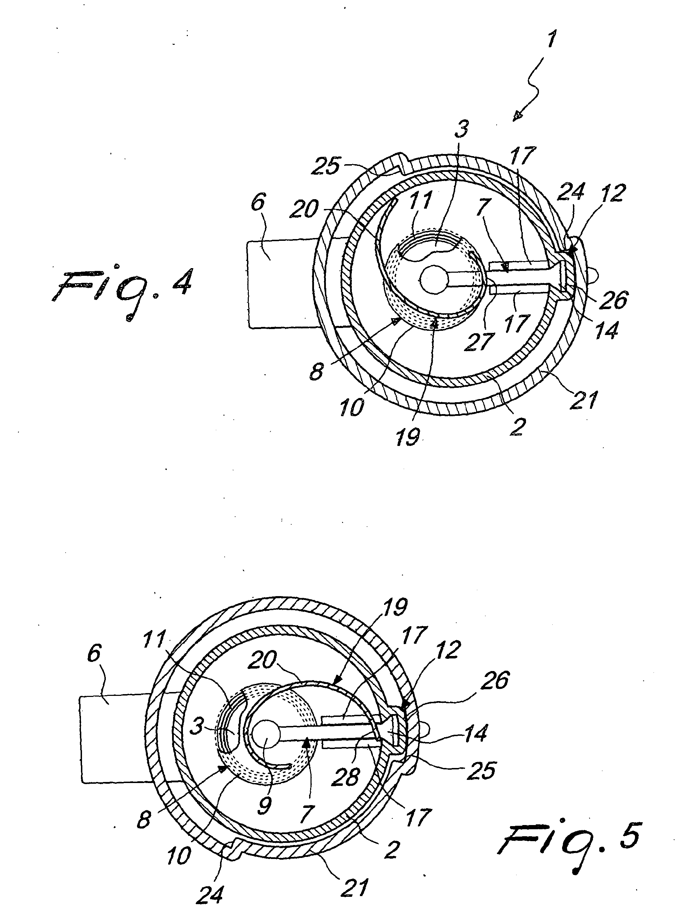

The rotation of the spiral wall 20 rigidly coupled to the annular element 21 and its particular spiral shape allow the movement of a line of contact between the spiral wall 20 and the flat spring 7 from a point of maximum pressure threshold, designated by the reference numeral 27 in Figure 4, in which the first tooth 24 makes contact with the protrusion 26, to a minimum pressure threshold point, designated by the reference numeral 28 in Figure 5, in which the second tooth 25 makes contact with the protrusion 26.

The valve 1 is provided with means for locking the end of the flat spring 7 which is rigidly associated with the hollow body 2.

In particular, the locking means are formed by the action of the spiral wall 20 in contrast with the sliding of the anchoring end 14 within the groove 13, preventing its exit from the opening 15.

In a possible alternative embodiment, the flat spring 7 has a variable transverse cross-section, which preferably increases from the end that is rigidly associated with the hollow body 2 to the end for supporting the flat element 10 so as to allow amplified adjustments of the pressure threshold value for minimal movements of the line of contact of the spiral wall 20 along the longitudinal axis of the flat spring 7.

The operation of the present invention is as follows.

The valve 1 is applied to assisted-ventilation equipment. In particular, it is connected by means of the intake opening 5 to a connector which is interposed between the delivery of an elastic bag and a mask or tracheal tube which can be applied to a patient.

The elastic bag is further provided with an intake which is connected to an external source for supplying a gaseous compound under pressure.

Before using the apparatus for the assisted ventilation of a patient, the value of the pressure threshold of the gaseous compound to be insufflated is adjusted by turning the annular element 21 with respect to the body 2 of the valve. This adjustment is performed according to the build, age and clinical condition of the patient.

Once the mask or tracheal tube has been applied to the patient, the gaseous compound is insufflated by means of a series of successive pressing actions applied to the bag by a doctor or health worker.

In the condition in which the pressure of the insufflated gaseous compound does not exceed the threshold value set on the valve 1 and defined as dangerous for the patient, the flat element 10 is engaged in the seat 11 and closes hermetically the intake port 3.

If the pressure value of the insufflated gaseous compound exceeds the threshold value set as maximum on the valve 1, the portion of the flat spring 7 that is formed between the transverse line of contact with the spiral wall 20 and the end for supporting the flat element 10 flexes, moving the flat element 10 away from the seat 11 and opening the intake port 3.

In this last case, the gaseous compound then enters from the intake opening 5, the stream passes through the intake port 3, continues within the hollow body 2, and exits from the vent 6 through the discharge port 4.

In practice it has been found that the described pressure limiting valve achieves the intended aim and objects, and with particular reference to its use for medical assisted-ventilation equipment, the fact is stressed that it allows safe use for the patient.

Further, the presence of the adjustment means described above allows accurate adjustment of the maximum pressure threshold value at which the valve opens, so as to adapt it precisely to the characteristics of the patient.

Finally, the execution of the flat spring and of the flow control element entirely by using plastic material ensures the use of the assisted-ventilation equipment even in combination with the use of diagnostic equipment which uses a magnetic field, such as for example magnetic resonance.

The invention thus conceived is susceptible of numerous modifications and variations, all of which are within the scope of the appended claims.

All the details may further be replaced with other technically equivalent elements.

In practice, the materials used, as well as the contingent shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A pressure limiting valve, particularly for medical assisted-ventilation equipment, comprising: a hollow body (2) provided with an intake port (3) and a delivery port (4) for a fluid; a flow control element (8) for controlling said intake port (3); a flat spring (7) which is accommodated within said hollow body, (2) arranged substantially at right angles to the input stream of the fluid and having a first end (14) which is rigidly associated with said hollow body (2), said flat spring (7) rigidly supporting said flow control element (8) and being flexible in the direction in which said flow control element (8) moves away from said intake port (3) at a presettable threshold value of the pressure applied by said fluid entering from said intake port (3), **characterized in that**, said flat spring (7) and said flow control element (8) are formed monolithically.

2. The valve according to claim 1, **characterized in that** said flat spring (7) is supported in a cantilever manner on said hollow body (2) at said first end (14) thereof, the flow control element (8) monolithically formed with said flat spring (7) being located proximate to a second free end thereof opposite to said first end (14).

3. The valve according to claims 1 or 2, **characterized in that** said flat spring (7) and said flow control element (8) are made of plastic material of the type that can be deformed elastically.

4. The valve according to claims 1 or 2, **characterized in that** said flat spring (7) and said flow control element (8) are made of silicone.

5. The valve according to claims 1 or 2, **characterized in that** said flat spring (7) and said flow control element (8) are made of polyurethane.

6. The valve according to one or more of the preceding claims, **characterized in that** said flat spring (7) has a tab (9) for supporting said flow control element (8).

7. The valve according to claim 6, **characterized in that** said tab (9) is constituted by a tubular element which is substantially perpendicular to the longitudinal axis of the flat spring (7).

8. The valve according to one or more of the preceding claims, **characterized in that** said flow control element (8) comprises a substantially circular flat element (10).

9. The valve according to one or more of the preceding claims, **characterized in that** it comprises means (12) for coupling said first end of said flat spring (7) to said hollow body (2).

10. The valve according to claim 9, **characterized in that** said coupling means comprise a dovetail prismatic guide (12) which is provided so as to run substantially longitudinally with respect to said hollow body (2).

11. The valve according to one or more of the preceding claims, **characterized in that** it comprises means (13) for locking said first end (14) of said flat spring (7) which is rigidly associated with said hollow body (2).

12. The valve according to claim 10 or 11, **characterized in that** it comprises two side walls (17) for containing said flat spring (7) which are formed so as to protrude from said guide (12) and are substantially parallel to each other and are laterally adjacent, along at least one portion, with respect to the two opposite sides of said flat spring (7).

13. The valve according to one or more of the preceding claims, **characterized in that** it comprises means (18) for adjusting said pressure threshold value.

14. The valve according to claim 13, **characterized in that** said adjustment means (18) comprise elements (19, 21) for adjusting the flexibility of said flat spring (7).

15. The valve according to claim 14, **characterized in that** said adjustment elements comprise an abutment element (19), which is adapted to make contact in at least one point or transverse line with the surface of said flat spring (7) that lies opposite said flow control element (8), said abutment element (19) being associated with said hollow body (2) so that it can move slidingly in the direction of the length of said flat spring (7).

16. The valve according to claim 15, **characterized in that** said abutment element (19) comprises an annular element (21), which is rotatably associated with said hollow body (2), is provided with a wall (20) which is substantially spiral-shaped, protrudes within said hollow body (2) and has a profile which is adapted to make contact with said surface of said flat spring (7), rotation of said annular element (21) with respect to said hollow body (2) determining a movement of a different portion of said profile into contact with said surface.

17. The valve according to claim 16, **characterized in that** it comprises means (26) for stopping the rotation of said annular element (21) in two maximum and minimum limit configurations of said pressure threshold value.

18. The valve according to claim 17, **characterized in that** said stop means comprise at least one protruding portion of the side wall of said annular element (21) and a first tooth (24) and a second tooth (25) which are formed at ends of said portion of the side wall of said annular element (21), said first and second teeth (24, 25) being adapted to make contact with a protrusion (26) formed on said hollow body (2) respectively in said maximum and minimum limit pressure threshold value configurations.

19. The valve according to one or more of claims 16 to 18, **characterized in that** said locking means comprise a groove surface (13) for supporting said first end (14) of the flat spring (7) that is coupled to said guide (12), said surface (13) being formed in the guide (12) and cooperating with said annular element (21), said spiral wall (20) being adapted to lock said first end (14) of said flat spring (7) in abutment against said supporting surface (13).

20. The valve according to one or more of the preceding claims, **characterized in that** said flat spring (7) has a substantially constant transverse cross-section.

21. The valve according to one or more of the preceding claims, **characterized in that** said flat spring (7) has a variable transverse cross-section.

22. The valve according to any of the claims 2-21, **characterized in that** said flat spring (7) has a transverse cross-section which increases from said first end (14) rigidly associated with said hollow body (2) to the free end for supporting said flow control element (8).

## Patentansprüche

1. Ein Druckbegrenzungsventil, insbesondere für medizinisches Gerät zur assistierten Beatmung, das Folgendes umfasst:
einen hohlen Körper (2), der mit einer Einiass-Öffnung (3) und einer Auslass-Öffnung (4) für eine Flüssigkeit ausgestattet ist;
ein Durchflussregelungselement (8) zur Steuerung der Einlass-Öffnung (3), eine Flachfeder (7), die in dem hohlen Körper (2) untergebracht ist, angeordnet im Wesentlichen in rechten Winkeln zum Eingangsstrom der Flüssigkeit, die ein erstes Ende (14) hat,
das starr mit dem hohlen Körper (2) verknüpft ist, wobei die Flachfeder (7) das Durchflussregelungselement (8) starr stützt und in der Richtung biegsam ist, in der sich das Durchflussregelungselement (8) von der Einlass-Öffnung (3) fort bewegt, bei einem voreinstellbaren Schwellenwert des Drucks, der von der Flüssigkeit ausgeübt wird, welche von der Finlass-öffnung (3) eintritt, **dadurch gekennzeichnet, dass** die Flachfeder (7) und das Durchflussregelungselement (8) monolithisch geformt sind.

2. Das Ventil gemäß Anspruch 1, **dadurch gekennzeichnet, dass** die Flachfeder (7) auf eine freitragende Art und Weise an dem hohlen Körper (2) an dem ersten Ende (14) davon getragen wird, wobei sich das Durchflussregelungselement (8), das monolithisch mit der Flachfeder (7) geformt ist, nahe einem zweiten freien Ende davon befindet, das dem ersten Ende (14) gegenüberliegt.

3. Das Ventil gemäß Anspruch 1 oder 2, **dadurch gekennzeichnet, dass** die Flachfeder (7) und das Durchflussregelungselement (8) aus Kunststoffmaterial von der Art bestehen, die elastisch verformt werden kann.

4. Das Ventil gemäß Anspruch 1 oder 2, **dadurch gekennzeichnet, dass** die Flachfeder (7) und das Durchflussregelungselement (8) aus Silikon hergestellt sind.

5. Das Ventil gemäß Anspruch 1 oder 2, **dadurch gekennzeichnet, dass** die Flachfeder (7) und das Durchflussregelungselement (8) aus Polyurethan hergestellt sind.

6. Das Ventil gemäß einem oder mehreren der obigen Ansprüche, **dadurch gekennzeichnet, dass** die Flachfeder (7) einen Lappen (9) zum Tragen des Durchflussregelungselements (8) hat.

7. Das Ventil gemäß Anspruch 6, **dadurch gekennzeichnet, dass** der Lappen (9) aus einem rohrförmigen Element besteht, das im Wesentlichen senkrecht zur Längsachse der Flachfeder (7) ist.

8. Das Ventil gemäß einem oder mehreren der obigen Ansprüche, **dadurch gekennzeichnet, dass** das Durchflussregelungselement (8) ein im Wesentlichen kreisförmiges flaches Element (10) umfasst.

9. Das Ventil gemäß einem oder mehreren der obigen Ansprüche, **dadurch gekennzeichnet, dass** es Mittel (12) umfasst, um das erste Ende der Flachfeder (7) mit dem hohlen Körper (2) zu verbinden.

10. Das Ventil gemäß Anspruch 9, **dadurch gekennzeichnet, dass** die Verbindungsmittel eine schwalbenschwanzförmige prismatische Führung (12) umfassen, die so bereitgestellt ist, dass sie im Wesentlichen im Längsrichtung zu dem hohlen Körper (2) verläuft.

11. Das Ventil gemäß einem oder mehreren der obigen Ansprüche, **dadurch gekennzeichnet, dass** es Mittel (13) zur Verriegelung des ersten Endes (14) der Flachfeder (7) umfasst, das starr mit dem hohlen Körper (2) verknüpft ist.

12. Das Ventil gemäß Anspruch 10 oder 11, **dadurch gekennzeichnet, dass** es zwei Seitenwände (17) zum Aufnehmen der Flachfeder (7) umfasst, die so geformt sind, dass sie aus der Führung (12) herausragen, und die im Wesentlichen parallel zueinander sind, und, entlang mindestens einem Abschnitt, an die zwei gegenüberliegenden Seiten der Flachfeder (7) seitlich angrenzen.

13. Das Ventil gemäß einem oder mehreren der obigen Ansprüche, **dadurch gekennzeichnet, dass** es Mittel (18) umfasst, um den Druck-Schwellenwert einzustellen.

14. Das Ventil gemäß Anspruch 13, **dadurch gekennzeichnet, dass** die Einstellmittel (18) Elemente (19, 21) umfassen, um die Biegsamkeit der Flachfeder (7) einzustellen.

15. Das Ventil gemäß Anspruch 14, **dadurch gekennzeichnet, dass** die Einstellelemente ein Widerlagerelement (19) umfassen, das ausgebildet ist, um in mindestens einem Punkt oder einer transversalen Linie die Oberfläche der Flachfeder (7) zu berühren, die gegenüber dem Durchflussregelungselement (8) liegt, wobei das Widerlager (19) mit dem hohlen Körper (2) so verknüpft ist, dass es sich verschiebbar in Richtung der Länge der Flachfeder (7) bewegen kann.

16. Das Ventil gemäß Anspruch 15, **dadurch gekennzeichnet, dass** das Widerlagerelement (19) ein ringförmiges Element (21) umfasst, das drehbar mit dem hohlen Körper (2) verknüpft ist, mit einer Wand (20) versehen ist, die im Wesentlichen spiralförmig ist, in den hohlen Körper (2) hineinragt und ein Profil hat, das ausgebildet ist, um die Oberfläche der Flachfeder (7) zu berühren, wobei die Drehung des ringförmigen Elements (21) in Bezug zu dem hohlen Körper (2) eine Bewegung eines anderen Abschnitts des Profils in Kontakt mit der Oberfläche bestimmt.

17. Das Ventil gemäß Anspruch 16, **dadurch gekennzeichnet, dass** es Mittel (26) umfasst, um die Drehung des ringförmigen Elements (21) in zwei maximalen und minimalen Grenz-Anordnungen des Druck-Schwellenwerts zu stoppen.

18. Das Ventil gemäß Anspruch 17, **dadurch gekennzeichnet, dass** die Stoppmittel Folgendes umfassen: mindestens einen vorstehenden Abschnitt der Seitenwand des ringförmigen Elements (21) und einen ersten Zahn (24) und einen zweiten Zahn (25), die an Enden des Abschnitts der Seitenwand des ringförmigen Elements (21) geformt sind, wobei der erste und der zweite Zahn (24, 25) ausgebildet sind, um in den maximalen beziehungsweise minimalen Grenz-Druck-Schwellenwert-Anordnungen einen Vorsprung (26) zu berühren, der an dem hohlen Körper (2) geformt ist.

19. Das Ventil gemäß einem oder mehreren der Ansprüche 16 bis 18, **dadurch gekennzeichnet, dass** die Verriegelungsmittel eine Rillenoberfläche (13) zum Tragen des ersten Endes (14) der Flachfeder (7) umfassen, das mit der Führung (12) gekoppelt ist, wobei die Oberfläche (13) in der Führung (12) geformt ist und mit dem ringförmigen Element (21) zusammenwirkt und die Spiralwand (20) ausgebildet ist, um das erste Ende (14) der Flachfeder (7) anstoßend an die tragende Oberfläche (13) zu blockieren.

20. Das Ventil gemäß einem oder mehreren der obigen Ansprüche, **dadurch gekennzeichnet, dass** die Flachfeder (7) einen im Wesentlichen konstanten transversalen Querschnitt hat.

21. Das Ventil gemäß einem oder mehreren der obigen Ansprüche, **dadurch gekennzeichnet, dass** die Flachfeder (7) einen variablen transversalen Querschnitt hat.

22. Das Ventil gemäß einem beliebigen der Ansprüche 2-21, **dadurch gekennzeichnet, dass** die Flachfeder (7) einen transversalen Querschnitt hat, der von dem ersten Ende (14), das starr mit dem hohlen Körper (2) verküpft ist, zu dem freien Ende zum Tragen des Durchflussregelungselements (8) hin zunimmt.

## Revendications

1. Valve de limitation de pression, en particulier pour équipements médicaux de ventilation assistée, comprenant : un corps creux (2) pourvu d'un orifice d'admission (3) et d'un orifice de refoulement (4) pour un fluide ; un élément de régulation (8) de débit pour commander ledit orifice d'admission (3) ; un ressort plat (7) logé dans ledit corps creux (2), disposé sensiblement perpendiculairement au courant d'entrée de fluide et ayant une première extrémité (14) qui fait corps avec ledit corps creux (2), ledit ressort plat (7) supportant d'une manière solidaire ledit élément de régulation (8) de débit et étant flexible dans la direction dans laquelle ledit élément de régulation (8) de débit s'écarte dudit orifice d'admission (3) à une valeur de seuil préréglable de la pression appliquée par ledit fluide entrant depuis ledit orifice d'admission (3), **caractérisée en ce que** ledit ressort plat (7) et ledit élément de régulation (8) de débit sont formés d'un seul tenant.

2. Valve selon la revendication 1, **caractérisée en ce que** ledit ressort plat (7) est supporté en porte-à-faux à ladite première extrémité (14) de celui-ci sur ledit corps creux (2), l'élément de régulation (8) de débit formé d'un seul tenant avec ledit ressort plat (7) étant situé tout près d'une seconde extrémité de celui-ci, opposée à ladite première extrémité (14).

3. Valve selon la revendication 1 ou 2, **caractérisée en ce que** ledit ressort plat (7) et ledit élément de régulation (8) de débit sont en matière plastique du type pouvant se déformer par élasticité.

4. Valve selon la revendication 1 ou 2, **caractérisée en ce que** ledit ressort plat (7) et ledit élément de régulation (8) de débit sont en silicone.

5. Valve selon la revendication 1 ou 2, **caractérisée en ce que** ledit ressort plat (7) et ledit élément de régulation (8) de débit sont en polyuréthane.

6. Valve selon une ou plusieurs des revendications précédentes, **caractérisée en ce que** ledit ressort plat (7) possède une patte (9) pour supporter ledit élément de régulation (8) de débit.

7. Valve selon la revendication 6, **caractérisé en ce que** ladite patte (9) est constituée par un élément tubulaire sensiblement perpendiculaire à l'axe longitudinal du ressort plat (7).

8. Valve selon une ou plusieurs des revendications précédentes, **caractérisée en ce que** ledit élément de régulation (8) de débit comporte un élément plat sensiblement circulaire (10).

9. Valve selon une ou plusieurs des revendications précédentes, **caractérisée en ce qu'**elle comprend un moyen (12) pour monter ladite première extrémité dudit ressort plat (7) sur ledit corps creux (2).

10. Valve selon la revendication 9, **caractérisée en ce que** ledit moyen de montage comporte un guide prismatique à queue d'aronde (12) permettant un mouvement sensiblement longitudinal par rapport audit corps creux (2).

11. Valve selon une ou plusieurs des revendications précédentes, **caractérisée en ce qu'**elle comprend un moyen (13) pour verrouiller ladite première extrémité (14) dudit ressort plat (7) qui fait corps avec ledit corps creux (2).

12. Valve selon la revendication 10 ou 11, **caractérisée en ce qu'**elle comprend deux parois latérales (17) pour contenir ledit ressort plat (7), qui sont formées de manière à faire saillie depuis ledit guide (12) et sont sensiblement parallèles l'une à l'autre et sont latéralement adjacentes, sur au moins une partie, par rapport aux deux côtés opposés dudit ressort plat (7).

13. Valve selon une ou plusieurs des revendications précédentes, **caractérisée en ce qu'**elle comprend un moyen (18) pour régler ladite valeur de seuil de pression.

14. Valve selon la revendication 13, **caractérisée en ce que** ledit moyen de réglage (18) comporte des éléments (19, 21) pour régler la flexibilité dudit ressort plat (7).

15. Valve selon la revendication 14, **caractérisée en ce que** lesdits éléments de réglage comportent un élément de butée (19), conçu pour venir au contact, en au moins un point ou une ligne transversale, de la surface dudit ressort plat (7) située en regard dudit élément de régulation (8) de débit, ledit élément de butée (19) étant associé audit corps creux (2) de façon à pouvoir se déplacer en coulissant dans le sens de la langueur dudit ressort plat (7).

16. Valve selon la revendication 15, **caractérisée en ce que** ledit élément de butée (19) comporte un élément annulaire (21) qui est associé d'une manière rotative audit corps creux (2), est pourvu d'une paroi (20) d'une forme sensiblement en spirale, fait saillie à l'intérieur dudit corps creux (2) et a un profil conçu pour venir au contact de ladite surface dudit ressort plat (7), la rotation dudit élément annulaire (21) par rapport audit corps creux (2) déterminant un mouvement d'une partie différente dudit profil venant au contact de ladite surface.

17. Valve selon la revendication 16, **caractérisée en ce qu'**elle comprend un moyen (26) pour arrêter la rotation dudit élément annulaire (21) dans deux configurations de limites maximale et minimale de ladite valeur de seuil de pression.

18. Valve selon la revendication 17, **caractérisée en ce** ledit moyen d'arrêt comporte au moins une partie saillante de la paroi latérale dudit élément annulaire (21) et une première dent (24) et une seconde dent (25) formées à des extrémités de ladite partie de la paroi latérale dudit élément annulaire (21), lesdites première et seconde dents (24, 25) étant respectivement conçues pour venir au contact d'une saillie (26) formée sur ledit corps creux (2)dans lesdites configurations à valeurs limites maximale et minimale de seuil de pression.

19. Valve selon une ou plusieurs des revendications 16 à 18, **caractérisée en ce que** ledit moyen de verrouillage comporte une surface cannelée (13) pour supporter ladite première extrémité (14) du ressort plat (7) monté sur ledit guide (12), ladite surface (13) étant formée dans le guide (12) et coopérant avec ledit élément annulaire (21), ladite paroi en spirale (20) étant conçue pour verrouiller ladite première extrémité (14) dudit ressort plat (7) en butée contre ladite surface de support (13).

20. Valve selon une ou plusieurs des revendications précédentes, **caractérisée en ce que** ledit ressort plat (7) a une section transversale sensiblement constante.

21. Valve selon une ou plusieurs des revendications précédentes, **caractérisée en ce que** ledit ressort plat (7) a une section transversale variable.

22. Valve selon l'une quelconque des revendications 2 à 21, **caractérisée en ce** ledit ressort plat (7) a une section transversale qui augmente de ladite première extrémité (14) faisant corps avec ledit corps creux (2) à l'extrémité libre pour supporter ledit élément de régulation (8) de débit.
